# EUROPEAN PATENT APPLICATION

(11) **EP 3 825 422 A2**
(43) Date of publication of application: **26.05.2021**
(21) Application number: 19838597.3
(22) Date of filing: 11.07.2019
(51) Int. Cl.: C12Q 1/689

(54) **DIAGNOSTIC KIT AND METHOD FOR THE SIMULTANEOUS DETECTION OF A COMBINATION OF MULTIPLE GRAM-POSITIVE BACTERIA AND/OR GRAM-NEGATIVE BACTERIA**

(30) Priority: 16.07.2018 MX 2018008742
(71) Applicant: Sigma Alimentos, S. A. De C. V., Nuevo León 66254 (MX)
(72) Inventor: RÍOS LICEA, Merab Magaly, Nuevo León, 66492 (MX); DE HAENE ROSIQUE, Gregorio José, Nuevo León, 64987 (MX); RIVAS ESTILLA, Ana María Guadalupe, Nuevo León, 64460 (MX)
(74) Representative: Codoñer Molina, Vicente
(86) International application number: PCT/MX2019/000084
(87) International publication number: WO 2020/017947

(57) **Abstract**

A method for the simultaneous detection of a combination of multiple Gram-positive bacteria and/or Gram-negative bacteria in a sample, the method comprising: providing DNA extracted from the sample; preparing a reaction mixture containing the DNA, one or more pairs of oligonucleotide primers having a sequence selected from the group consisting of SEQ ID NO: 1 and SEQ ID NO: 2, SEQ ID NO: 4 and SEQ ID NO: 5, SEQ ID NO: 7 and SEQ ID NO: 8, SEQ ID NO: 10 and SEQ ID NO: 11, SEQ ID NO: 13 and SEQ ID NO: 14, SEQ ID NO: 16 and SEQ ID NO: 17 and combinations of same, and one or more oligonucleotide probes having a sequence selected from the group consisting of SEQ ID NO: 3, SEQ ID NO: 6, SEQ ID NO: 9, SEQ ID NO: 12, SEQ ID NO: 15, SEQ ID NO: 18 and combinations of same; amplifying the DNA included in the reaction mixture, by means real-time polymerase chain reaction; and simultaneously determining the presence or absence of Gram-positive bacteria and/or Gram-negative bacteria in the sample. The method can be used to identify any combination of three species of Gram-positive bacteria *(Listeria ssp., Listeria monocytogenes* and *Staphylococcus aureus)* and three species of Gram-negative bacteria *(Escherichia coli* spp., *Escherichia coli* O157:H7 and *Salmonella* spp.).

## Description

### TECHNICAL FIELD OF THE INVENTION

The present invention relates, in general, to the detection and identification of pathogenic bacteria, and particularly to a method and a kit for detecting multiple and simultaneously any combination of Gram-positive and/or Gram-negative bacteria, from among *Listeria* ssp., *Listeria monocytogenes, Staphylococcus aureus, Escherichia coli* spp., *Escherichia coli* O157:H7 y *Salmonella* spp., by multiplex amplification reaction, using real-time polymerase chain reaction.

### BACKGROUND OF THE INVENTION

At present, the detection of pathogenic bacteria transmitted by food constitutes a very important task in the field of medicine and public hygiene and is of great interest in the agri-food industry, both the producer and the distributor of food products (raw materials and/or manufactured products), for which various methods have been described for their detection and identification.

One of the current methodologies, considered among the most effective for the detection and identification of pathogens, is one that is based on molecular techniques, such as the polymerase chain reaction method, commonly known as PCR (for its acronym in English). The PCR technique is generally considered the most sensitive and rapid methodology used to detect nucleic acids, including genomes of pathogens in a particular test sample, and we can find it described within the state of the art by Kary B. Mullis et al.. in the family of US patents US-4683195, US-4683202, US-4800159, US-4889818, US-4965188, US-5008182, US-5038852, US-5079352, US-5176995, US-5310652, US-5310893, US-5322770, US-5333675, US-5352600, US-5374553, US-5386022, US-5405774, US-5407800, US-5418149, US-5420029 among others.

In order to carry out the PCR technique, it is basically necessary to have at least one pair of oligonucleotide primers specific for each of the pathogens to be identified, such that each pair of primers comprises a first unique nucleotide sequence, specific and complementary to a sequence that borders the 5'-end of a target nucleic acid sequence and a second nucleotide sequence unique, specific and complementary to a sequence that borders the 3'-end of the target nucleic acid sequence. The nucleotide sequences must have each pair of oligonucleotide primers that are specific to the pathogen to be detected, such that they do not react or cross-hybridize with other pathogens.

As the PCR technique is a specific, sensitive and rapid method to detect the genome of bacterial pathogens individually, this technique can also be used to simultaneously detect multiple pathogens present in a sample, directly or after enrichment in a selective medium.. However, using the PCR technique for the simultaneous detection of multiple pathogens in a sample has its problems, since its main obstacle lies in the reaction or cross hybridization that may occur with other pathogens due to the use of multiple nucleotide sequences in order to have the preferential amplification of certain target sequences present in the sample at the expense of other target sequences also present.

Examples of applications for multiple and simultaneous detection of pathogens, using the PCR technique, are described by John W. Czajka in the international patent application publication WO-2003014704, by Linxian Wo et al. in the family of US patents US-5612473, US-5738995, US-5753444, US-5756701 and US-5846783, by Gary Lawrence Frech et al. in US patent US-9109260.

In the international patent application publication WO-2003014704 a method is described for specifically and simultaneously detecting pathogenic *Campylobacter* species in a complex test sample. The pathogenic Campylobacter species to detect can be *Campylobacter jejuni* or *Campylobacter coli.* The complex test sample can be a food sample, water or a food enriched matrix. The method uses PCR amplification with or without an internal positive control and appropriate primer pairs. It is shown that multiple species can be detected in the same reaction.

While in the family of US patents US-5612473, US-5738995, US-5753444, US-5756701 and US-5846783 a multiplex PCR method is described to rapidly and simultaneously detect infectious agents in a sample. The infectious agents detected are *Salmonella* spp., *Shigella* spp., *Campylobacter* spp., *Yersinia* spp. and *Escherichia coli* in particular *Escherichia coli* O157:H7. The limitation of the method described in these patents is that it allows minimal cross-reaction between the oligonucleotides and probes, as well as these with other nucleic acid sequences during amplification.

On the other hand, US patent US-9109260 describes a method to identify one or more bacteria, from among *Escherichia coli, Staphylococcus aureus, Pseudomonas aeruginosa, Enterococeus* spp., *Klebsiella* spp., *Enterobacter* spp., *Proteus* spp., *Pneumococci,* and *Staphylococci* coagulase negative, present in a sample, starting from the amplification of the 23s ribosomal RNA subunit present in the sample using the PCR technique and a pair of primers, and then testing the amplification by selective hybridization of the amplification with probes of tests designed for each pathogen to be identified.

Based on the above, it is of great interest for the food and health industry to have a method and a diagnostic kit for detecting multiple and simultaneously, by multiplex amplification reaction using the polymerase chain reaction in real time, a combination of three of the most important Gram-positive bacteria (such as *Listeria* ssp., *Listeria monocytogenes* and *Staphylococcus aureus)* and/or three of the most important Gram-negative bacteria (such as *Escherichia coli* spp., *Escherichia coli* O157: H7 and *Salmonella* spp.) transmitted by food and /or contaminated environmental surfaces. This multiple and simultaneous detection of a combination of Gram-positive bacteria and/or Gram-negative bacteria allows to reduce at least the following limitations: (i) analysis costs per pathogen (obtaining multiple results in the same sample), (ii) processing time and the issuance of qualitative results (approximately 26 hours), which impact on the reduction of the shelf time of products in warehouses, and (iii) allow the release of the safe food product to the market in less time than the conventional one.

### SUMMARY OF THE INVENTION

Based on the above and with the purpose of solving the limitations found, it is the object of the invention to offer a method for detecting multiple and simultaneously a combination of Gram-positive bacteria and/or Gram-negative bacteria in a sample, the method has the steps of: a) providing DNA extracted from the sample; b) preparing a reaction mixture including: (i) the DNA provided; (ii) one or more pairs of oligonucleotide primers of selected sequence from the group consisting of SEQ ID NO: 1 and SEQ ID NO: 2, SEQ ID NO: 4 and SEQ ID NO: 5, SEQ ID NO: 7 and SEQ ID NO: 8, SEQ ID NO: 10 and SEQ ID NO: 11, SEQ ID NO: 13 and SEQ ID NO: 14, SEQ ID NO: 16 and SEQ ID NO: 17 and combinations thereof; and (iii) one or more oligonucleotide probes of selected sequence from the group consisting of SEQ ID NO: 3, SEQ ID NO: 6, SEQ ID NO: 9, SEQ ID NO: 12, SEQ ID NO: 15, SEQ ID NO: 18 and combinations thereof; c) amplifying, by means of real-time polymerase chain reaction (PCR), the DNA provided in the reaction mixture; and d) simultaneously determining the presence or absence of Gram-positive bacteria and/or Gram-negative bacteria in the sample.

Another aspect of the present invention consists in providing a diagnostic kit for detecting multiple and simultaneously a combination of Gram-positive bacteria and Gram-negative bacteria in a sample, the diagnostic kit includes one or more pairs of oligonucleotide primers of selected sequence from the group consisting of SEQ ID NO: 1 and SEQ ID NO: 2, SEQ ID NO: 4 and SEQ ID NO: 5, SEQ ID NO: 7 and SEQ ID NO: 8, SEQ ID NO: 10 and SEQ ID NO: 11 , SEQ ID NO: 13 and SEQ ID NO: 14, SEQ ID NO: 16 and SEQ ID NO: 17 and combinations thereof; and one or more oligonucleotide probes of selected sequence from the group consisting of SEQ ID NO: 3, SEQ ID NO: 6, SEQ ID NO: 9, SEQ ID NO: 12, SEQ ID NO: 15, SEQ ID NO: 18 and combinations thereof.

The diagnostic method and kit allow the detection of one or more of the following pathogens: *Listeria* ssp., *Listeria monocytogenes, Staphylococcus aureus, Escherichia coli* spp., *Escherichia coli* O157: H7 y *Salmonella* spp.

### BRIEF DESCRIPTION OF THE FIGURES

The characteristic details of the invention are described in the following paragraphs in conjunction with the accompanying figures, which are for the purpose of defining the invention but without limiting its scope.
Figure 1 illustrates a graph of dilutions of *Listeria* ssp., where A1 is a reference with the highest concentration and A4 is the reference with the lowest concentration. The reading of a sample is interpolated in the readings of the references to know the CFU/ml.
Figure 2 illustrates a graph of dilutions of *Listeria monocytogenes,* where A1 is a reference with the highest concentration and A4 is the reference with the lowest concentration. The reading of a sample is interpolated in the readings of the references to know the CFU/ml.
Figure 3 illustrates a graph of *Staphylococcus aureus* dilutions, where A1 is a reference with the highest concentration and A4 is the reference with the lowest concentration. The reading of a sample is interpolated in the readings of the references to know the CFU/ml.
Figure 4 illustrates a graph of dilutions of *Escherichia coli* spp., where A1 is a reference with the highest concentration and A4 is the reference with the lowest concentration. The reading of a sample is interpolated in the readings of the references to know the CFU/ml.
Figure 5 illustrates a graph of dilutions of *Escherichia coli* O157:H7, where A1 is a reference with the highest concentration and A4 is the reference with the lowest concentration. The reading of a sample is interpolated in the readings of the references to know the CFU/ml.
Figure 6 illustrates a graph of dilutions of *Salmonella* spp., where A1 is a reference with the highest concentration and A4 is the reference with the lowest concentration. The reading of a sample is interpolated in the readings of the references to know the CFU/ml.

### DETAILED DESCRIPTION OF THE INVENTION

The use of the word "a" or "an" when used in conjunction with the term "comprise" or "comprises" in the claims and/or the description may mean "one", but is also consistent with the meaning of "one or more", "at least one" and "one or more than one".

The term "enzymatic DNA amplification", as used in the context of the present description, means the use of the polymerase chain reaction (PCR) to increase the concentration of a particular DNA sequence within a mixture of DNA sequences. The particular DNA sequence that is amplified is referred to as a "target sequence."

In the present invention, "fluorophore" is understood as a molecule capable of emitting electromagnetic radiation in response to the absorption of excitation radiation, where the wavelength of the radiation emitted is different than the wavelength of the radiation from excitation and where the emission of radiation persists only as long as the excitation radiation is maintained.

In the present invention, "extinguisher" is understood to mean the molecule that accepts energy from a fluorophore and dissipates it in the form of heat or fluorescence.

The term "PCR" stands for polymerase chain reaction by which millions of copies of the desired DNA regions can be obtained. It is characterized by the use of primer pairs that delimit the region of which millions of copies will be made, which is also known as "enzymatic DNA amplification" during PCR. PCR is made up of a certain number of cycles, in turn made up of three phases in which the DNA strands separate, the primers join and the new DNA strands elongate. In each cycle, if the efficiency of the reaction is 100%, an exponential growth of the DNA fragments object of the amplification occurs.

The term "primer pair" is used under the meaning of a pair of oligonucleotide primers that are complementary to the sequences that border the target sequence. The primer pair consists of an "upstream" primer that has a nucleic acid sequence that is complementary to an "upstream" sequence of the target sequence, and a "downstream" primer that has a nucleic acid sequence that is complementary to a sequence "downstream" of the target sequence.

The term "multiplex amplification reaction" is understood as the polymerase chain reaction (PCR) in which more than one DNA sequence is amplified in the same reaction, by using two or more pairs of primers in a single tube along with the rest of the reaction reagents in order to simultaneously amplify multiple DNA sequences.

The term "real-time PCR reaction" is basically a conventional PCR reaction in which the amplification equipment (thermal cyclers) incorporate a fluorescence detection system, said detection being based on the use of specific molecules called fluorophores and extinguisher.

The term "oligonucleotide" refers to the sequence of nucleotide bases linked by phospho-diester links, usually no greater than 50 nucleotides.

In the present invention, "initiator" or "primer" is understood as the nucleotide sequence from which DNA polymerase initiates the synthesis of a new DNA molecule. Primers are short nucleotide sequences, approximately 15-26 nucleotides in length that can be aligned with a target DNA strand through base complementarity to form a hybrid between the primer and the target DNA strand. The DNA polymerase enzyme can then extend the primer along the target DNA strand.

In the context of the present invention, "TaqMan probe" is understood as hydrolysis probes designed to increase the specificity and sensitivity of the PCR technique. The TaqMan probe relies on the 5'-3 'exonuclease activity of Taq polymerase to cleave a labeled probe already hybridized to the target sequence. This cleavage of the probe allows the emission of fluorescence, which allows obtaining a quantitative measure of the accumulation of the product during the PCR cycles. The great advantage of the TaqMan probe is that it significantly increases the specificity of detection. Therefore, the fluorescence detected in the PCR thermal cycler is directly proportional to that of the released fluorophore and the amount of template DNA present in the PCR. Each TaqMan probe is designed to hybridize to a specific region of DNA that is to be amplified by a specific oligonucleotide primer pair. As Taq polymerase synthesizes the 3'-5 'sense strand, the 5'-3' exonuclease activity of this same enzyme degrades the TaqMan probe already hybridized to DNA. Degradation of the probe separates the fluorophore, thus breaking the proximity between it and the extinguisher, thus allowing the emission of fluorescence.

In the context of the present invention, "sample" is understood to be any material capable of containing DNA with a degree of purity that prevents its degradation or that causes inhibition in the PCR reaction.

In the context of the present invention, "aliquot" is understood as the part taken from an initial volume (liquid aliquot) or from a mass (solid aliquot), to be used in a laboratory test, whose physical properties and Chemicals, as well as their composition, represent those of the original substance. Normally the aliquots are the result of dividing an initial volume in several equal parts.

In the present invention, a combination of three Gram-positive bacteria and/or three Gram-negative bacteria are detected multiply and simultaneously by real-time PCR in one reaction or in several consecutive reactions, depending on the capacity of the thermocycler apparatus used, from DNA extracted from food samples, surfaces and environments that could be contaminated with these individual bacteria and/or in combination, enriched and unenriched. Among the Gram-positive bacteria to detect and identify we have *Listeria* ssp., *Listeria monocytogenes, Staphylococcus aureus* and their combinations, and among the Gram-negative bacteria to detect and identify we have *Escherichia coli* spp., *Escherichia coli* O157:H7, *Salmonella* spp. and comninations thereof.

The test sample can be any sample containing DNA in which it is desired to know the possible contamination by said Gram-positive and/or Gram-negative bacteria. In a particular embodiment, said test sample is a sample of a food product, for example, meat and dairy products, or a sample of contaminated surfaces or environments.

The present invention relates, in a first section, to a method for detecting multiple and simultaneously a combination of Gram-positive bacteria *(Listeria* ssp., *Listeria monocytogenes* and *Staphylococcus aureus)* and/or Gram-negative bacteria *(Escherichia coli* spp., *Escherichia coli* O157:H7 and *Salmonella* spp.) in a sample, the method comprises the steps of:
a) providing DNA extracted from the sample;
b) preparing a reaction mixture, wherein the reaction mixture includes:
   the DNA provided;
   one or more pairs of oligonucleotide primers of selected sequence from the group consisting of SEQ ID NO: 1 and SEQ ID NO: 2, SEQ ID NO: 4 and SEQ ID NO: 5, SEQ ID NO: 7 and SEQ ID NO: 8, SEQ ID NO: 10 and SEQ ID NO: 11, SEQ ID NO: 13 and SEQ ID NO: 14, SEQ ID NO: 16 and SEQ ID NO: 17 and combinations thereof; and
   one or more oligonucleotide probes of sequence selected from the group consisting of SEQ ID NO: 3, SEQ ID NO: 6, SEQ ID NO: 9, SEQ ID NO: 12, SEQ ID NO: 15, SEQ ID NO: 18 and combinations thereof;
c) amplifying, by means of real-time PCR, the DNA provided in the reaction mixture; and
d) simultaneously determining the presence or absence of Gram-positive bacteria and/or Gram-negative bacteria in the sample.

### PREPARATION AND EXTRACTION OF DNA FROM A SAMPLE

In a first step, the method of the present invention comprises the step of providing DNA extracted from a sample, for which it is necessary to take the sample and extract the DNA from it.

Sampling must be done with the appropriate protocol and methodology depending on the characteristics of the sample, that is, if it is a liquid, solid, frozen, lyophilized etc. The collection of the sample must be carried out avoiding any external contamination, both environmental and handling, to ensure its integrity and reliable results. It is necessary to use clean, dry, leak-free, wide-mouth, sterile containers of an appropriate size for the sample. Likewise, the storage conditions, the transport, the time between the collection of the sample and its delivery to the laboratory, as well as the performance of the analysis influence the results obtained since the microbial population can undergo qualitative and quantitative changes. All these considerations are widely known to those skilled in the art, and the protocols and procedures for collecting samples are clearly standardized and established by the competent authorities.

Any sample likely to be contaminated with a combination of *Listeria* ssp., *Listeria monocytogenes, Staphylococcus aureus, Escherichia coli* spp., *Escherichia coli* O157:H7 y *Salmonella* spp., Can be analyzed with the method of the present invention. In a particular embodiment, the sample can be a food sample or an environment sample, the food samples can be obtained from dairy products, meat products, prepared meals, frozen foods, beverages and their combinations; while ambient samples can be obtained from metal or plastic surfaces. The protocol and methodology for collecting each of the samples from these products or environments are widely known and routine practice for those skilled in the art.

Once the sample is obtained, it must be processed to obtain the DNA from it. DNA extraction can be carried out using any method known to those skilled in the art, for example for samples enriched in SEL medium DNA extraction can be carried out with chelating resins (Chelex 100) or ion exchange, beads of glass and mechanical disruption, or commercial extraction kits may be used, for example the "Q-Biogene fast DNA kit" or the "QIAamp (R) DNA Blood Mini Kit" (Qiagen, Hilden, Germany) or the "G-Spin IIp "(Intron Biotechnology, Korea) or the "Fast Prep System Bio 101" (Qbiogene, Madrid, Spain) or PrepMan Ultra (Invitrogen, USA).

Finally, with the concentration of DNA extracted, a suspension is formed that is heated to approximately 65°C, which causes a rapid dissolution of the DNA sample.

### DESIGN AND SEQUENCE INFORMATION OF OLIGONUCLEOTIDE INITIATORS AND PROBES

To develop the method of the present invention, TaqMan primers and probes were designed to amplify DNA sequences specific to each microorganism of interest. For this, genetic targets with high specificity and conservation were selected. The nucleotide sequences of DNA and proteins corresponding to these targets were obtained from the NCBI (National Center for Biotechnology Information) database: GenBank and Primer BLAST. Alignments between these and the rest of the sequences deposited in the GeneBank were subsequently carried out using the Amplify 4.0 and BLAST (NCBI) applications, available on the digital page. The regions corresponding to conserved domains between strains of the same species were selected for the design of the primers, which was carried out with the Amplify 4.0 and BLAST (NCBI) application, in the case of real-time PCR

All initiators were synthesized in vitro and dissolved in water at room temperature. The TaqMan probes were synthesized in vitro, resuspended in water and kept at -20° C until use.

The oligonucleotides of the invention have been designed with the purpose of specifically identifying *Listeria* ssp., *Listeria monocytogenes, Staphylococcus aureus, Escherichia coli* spp., *Escherichia coli* O157:H7 y *Salmonella* spp., Without obtaining false positive results for detection of other microorganisms present in the same sample.

The activities that make up the design protocol of the primers and probes were the following:
1) Search for genes used for specific detection of each microorganism, in indexed journal publications.
2) Selection of several target genes used and search of the corresponding sequences in GenBank.
3) Selected target gene sequences are screened for primer and probe candidates using Primer Blast (NCBI) and Primer Quest tool (IDT).
4) Verification of primers and probes (Tm,% GC, homodimers, heterodimers and hairpins) using the Oligo Analyzer (IDT) and Amplify tools.
5) Verification of specificity of initiators and candidate probes in silica, using the Primer Blast tool.
6) Verification of initiators and probes (heterodimers) included in each reaction cocktail.

There is a pair of oligonucleotide primers for each of the Gram-positive bacteria *(Listeria* spp., *Listeria monocytogenes y Staphylococcus aureus)* and Gram-negative *(Escherichia coli* spp., *Escherichia coli* O157:H7 y *Salmonella* spp.) to be identified, such that each primer pair comprises a specific "upstream" synthetic nucleotide sequence complementary to an "upstream" nucleotide sequence that borders the 5'-end of a target nucleic acid sequence and a second "downstream" synthetic nucleotide sequence complementary to a "downstream" sequence that borders the 3'-end of the target nucleic acid sequence. The nucleotide sequences must have each pair of oligonucleotide primers that are specific to the Gram-positive or Gram-negative bacteria to be detected, in such a way that they do not react or cross with other microorganisms. Likewise, for each of the Gram-positive or Gram-negative bacteria to be identified, a synthetic sequence was developed that functions as a TaqMan probe. The primer sequences, tagging fluorophore probes, and selected target genes for each microorganism of interest are shown in Table 1.

**Table 1**

| Identifier | Type | Bacteria | Nucleotide Sequence 5' - 3' | Target gen |
|---|---|---|---|---|
| SEQ ID NO: 1 | Initiator "upstream" | *Listeria* ssp. | ATAGGGAATCGCACGAATGG | Consensus sequence of gene 23s |
| SEQ ID NO: 2 | Initiator "downstream" | | AGCGGATTTGCCTACTTCTC | |
| SEQ ID NO: 3 | Probe | | AAATGTGCGTCCAAGCAGTGAGTG | |
| SEQ ID NO: 4 | Initiator "upstream" | *Listeria monocytogenes* | CTCATTTCACATCGTCCATCTA | hyla |
| SEQ ID NO: 5 | Initiator "downstream" | | GTTCTCCACCATTCCCAAG | |
| SEQ ID NO: 6 | Probe | | TGTTTACGCTAAAGAATGCACTGGT | |
| SEQ ID NO: 7 | Initiator "upstream" | *Staphylococcus aureus* | TGATACACCTGAAACAAAGCATCCT | nuc |
| SEQ ID NO: 8 | Initiator "downstream" | | GACTTCAATTTTCTTTGCATTTTCTACCATTTT | |
| SEQ ID NO: 9 | Probe | | CAGGGCCATATTTCTC | |
| SEQ ID NO: 10 | Initiator "upstream" | *Escherichia coli* spp. | GGCAAAGTGTGGGTCAATA | beta-D-glucuronidase |
| SEQ ID NO: 11 | Initiator "downstream" | | GATAGTCTGCCAGTTCAGTTC | |
| SEQ ID NO: 12 | Probe | | AAGCCGATGTCACGCCGTATGTTA | |
| SEQ ID NO: 13 | Initiator "upstream" | *Escherichia coli* 0157:H7 | CTTACGCTTCAGGCAGATACAGA | Shiga type II toxin |
| SEQ ID NO: 14 | Initiator "downstream" | | CACAGGAGCAGTTTCAGACAGT | |
| SEQ ID NO: 15 | Probe | | CCTGACGAAATTCTC | |
| SEQ ID NO: 16 | Initiator "upstream" | *Salmonella* spp. | GTCCTAACGACGACCCTTCTTTT | invA |
| SEQ ID NO: 17 | Initiator "downstream" | | GCCAAACCTAAAACCAGCAAAGG | |
| SEQ ID NO: 18 | Probe | | CAGCCGCTCAGTATTG | |

### DESIGN AND SEQUENCE INFORMATION FOR INTERNAL AMPLIFICATION CONTROL

The PCR technique for the detection of pathogens, especially in food, can be affected by the presence of substances present in the samples with the capacity to inhibit the Taq polymerase enzyme present in the reaction. For this reason, in the present invention, an internal control amplification (CIA) DNA probe was designed that can be co-amplified, in such a way that it can be ensured that a negative result with the microorganism-specific probe is not due to inhibition of Taq polymerase, if not in the absence of complementarity between the probe and the sequence or in the absence of amplification due to the absence of binding of the primers.

The advantages of an internal amplification control contained in the PCR reaction are described in international patent application publication WO1997011197, the contents of which are incorporated herein by reference, and include (i) the control can be amplified using a single primer; (ii) the amount of the control amplification product is independent of any DNA target contained in the sample; (iii) the control DNA can be compressed with other amplification reagents for ease of use and high degree of reproducibility in manual and automated test procedures; (iv) the control can be used with homogeneous detection, that is, without separation of the product DNA from the reagents and (v) the internal control has a melting profile that is distinct from other potentially produced initiators in the reaction. Control DNA will be of the appropriate size and base composition to allow amplification in a primer directed amplification reaction. The control DNA sequence can be obtained from bacterial targets, or from another source, but must be reproducibly amplified under the same conditions that allow for amplification of the target primer DNA. The control reaction is useful to validate the amplification reaction. Control DNA amplification occurs within the same reaction tube as the sample being tested, and therefore indicates a successful amplification reaction when samples are target negative, that is, no target amplification occurs. To achieve meaningful validation of the amplification reaction, an adequate number of copies of the control DNA must be included in each amplification reaction.

The internal control amplification sequences developed in the present invention are described below in Table 2.

**Table 2**

| Identifier | Type | Internal amplification control | Nucleotide Sequence 5' - 3' | Target gen |
|---|---|---|---|---|
| SEQ ID NO: 19 | Initiator "upstream " | CIA | CCGACCAAATCCAACCAGTAA | UFGT3 |
| SEQ ID NO: 20 | Initiator "downstream" | | CTGGAGAGGATGAGTGAGAAGT | |
| SEQ ID NO: 21 | Probe | | CCAAAGCCACCTGAACCAACTCCT | |

### PREPARING THE REACTION MIXTURE

A multiplex amplification reaction requires a series of reagents, including, but not limited to, the extracted DNA, the DNA polymerase enzyme, at least two primer pairs (each primer in each pair being specific and complementary to one of the two strands of DNA), deoxynucleotide triphosphates (dNTPs), magnesium chloride (MgCl2), reaction buffer and optional additives, which can be added separately by mixing in the laboratory or purchased previously mixed, such as Thermo Fisher, Qiagen Multiplex PCR Kit (Qiagen, Valencia, CA) among others, to which the primer pairs in the appropriate concentrations and the provided DNA are added. The PCR technique is carried out in a thermal cycler that cycles at precisely programmed times and temperatures, such as the hybridization temperature, which depends on the melting temperature of each of the initiators used in the reaction or extension temperature.

### MULTIPLEX AMPLIFICATION REACTION USING REAL-TIME PCR

In a preferred embodiment of the invention, the multiplex amplification reaction employing real-time PCR provides many advantages over the conventional single-target-to-detect PCR method. The multiplex amplification reaction using PCR requires the development of oligonucleotide primers and TaqMan probes specific for the target sequence of the Gram-positive or Gram-negative bacteria to be detected, in such a way that said oligonucleotide primers and probes are compatible with each other within the same optimum temperature between 42°C to 65°C and subjected to the same chemical reaction conditions in order to allow the pairing or hybridization of two complementary nucleic acid segments. In addition to this, the oligonucleotide primer kits and probes must not react or cross or hybridize with each other, during amplification, to other nucleic acid sequences for which they were not designed.

The multiplex amplification reaction using PCR is carried out in an apparatus that is capable of varying the incubation temperature in the same block in a range of 37 - 94 °C, which generally consists of a thermal cycler with a plurality of holes in which tubes containing the reaction mixture for enzymatic DNA amplification are introduced; a light source coupled to the thermal cycler and adapted to distribute the light over the plurality of holes; and a fluorescence sensor or detector adapted to simultaneously detect the emitted light. In addition to this, there are currently numerous thermal cyclers with technological improvements that allow detecting changes through the sets of fluorophores and extinguisher used in the probes and initiators.

### DETECTION OF GRAM-POSITIVE OR GRAM-NEGATIVE BACTERIA

After completing the multiplex amplification reaction, the presence or absence of Gram-positive or Gram-negative bacteria in the sample is simultaneously determined. For which, in the present invention, the initiators and probes that participate in said multiplex amplification reaction were previously marked in order to subsequently detect the amplified fragments directly by means of laser detectors that are part of the real-time PCR thermocycler equipment. Labeling of the amplification products can be carried out by conventional methods. Said labeling can be direct, for which fluorophores, fluorescein, alexa, etc., enzymes, for example, alkaline phosphatase, peroxidase, etc., radioactive isotopes, for example, 33P, 1251, etc., or any other marker known to the person skilled in the art.

Examples of fluorescent materials that can be employed in oligonucleotide labeling include, but are not limited to, 5-carboxyfluorescein (5-FAM), 6-FAM, tetrachlorinated analog of t-FAM (TET), hexachlorinated analog of 6-FAM (HEX), 6-carboxytetramethylrhodamine (TAMRA), 6-carboxy-X-rhodamine (ROX), 6-carboxy-4,5-dichloro-2,7-dimethoxyfluorescein (JOE), NED, Cy-3, Cy-5, Cy -5.5, fluorescein-6-isothiocinate (FITC) and tetramethylrhodamine-5-isothiocinate (TRUC).

Fluorophores and extinguisher used as probes markers to detect and identify *Listeria* ssp., *Listeria monocytogenes, Staphylococcus aureus, Escherichia coli* spp., *Escherichia coli* O157:H7 y *Salmonella* spp., without producing cross reactions between them and the other components, are shown in Table 3. The VIC, FAM and JUN fluorophores were selected for the labeling of the Gram-positive and Gram-negative bacteria in the study according to real cases in which the simultaneous presence of Gram-positive and Gram-negative bacteria has been detected in the same sample and on the basis that the best fluorescence emission results were obtained. Examples: *Listeria* spp. (VIC) + *Listeria monocytogenes* (FAM) + *Salmonella* spp. (JUN). *Listeria monocytogenes* (FAM) + *Staphylococcus aureus* (JUN) + *Escherichia coli* spp. (VIC).

**Table 3**

| Identifier | Type | Bacteria/CIA | Nucleotide Sequence 5' - 3' | Fluorophor for 5'-end marking | Extinguisher for 3'-end marking |
|---|---|---|---|---|---|
| SEQ ID NO: 3 | Probe | *Listeria* ssp. | AAATGTGCGTCCAAGCAGTGAGTG | VIC | MGB |
| SEQ ID NO: 6 | Probe | *Listeria monocytogenes* | TGTTTACGCTAAAGAATGCACTGGT | FAM | MGB |
| SEQ ID NO: 9 | Probe | *Staphylococcus aureus* | CAGGGCCATATTTCTC | JUN | QSY |
| SEQ ID NO: 12 | Probe | *Escherichia coli* spp. | AAGCCGATGTCACGCCGTATGTTA | VIC | MGB |
| SEQ ID NO: 15 | Probe | *Escherichia coli* 0157:H7 | CCTGACGAAATTCTC | FAM | MGB |
| SEQ ID NO: 18 | Probe | *Salmonella* spp. | CAGCCGCTCAGTATTG | JUN | QSY |
| SEQ ID NO: 21 | Probe | CIA | CCAAAGCCACCTGAACCAACTCCT | ANY | BHQ-2 |

The absence or presence of Gram-positive bacteria and/or Gram-negative bacteria in any combination of: *Listeria* ssp., *Listeria monocytogenes, Staphylococcus aureus, Escherichia coli* spp., *Escherichia coli* O157:H7 y *Salmonella* spp. in a sample, it is determined by a fluorescent signal or fluorescence emission specific to each amplified DNA product or sample. For this, a specific wavelength is impinged for the excitation of each fluorophore and its emission at specific wavelengths is detected.

As previously explained, the method of the invention comprises the detection of up to 6 pathogenic microorganisms by means of one or more multiplex PCR reactions depending on the capacity of the thermal cycler apparatus used. However, DNA extraction and detection can be carried out from an unenriched food sample, surface or environment, artificially contaminated with the microorganisms of interest in different combinations, or in samples artificially contaminated with individual microorganisms or in combinations and enriched in SEL medium.

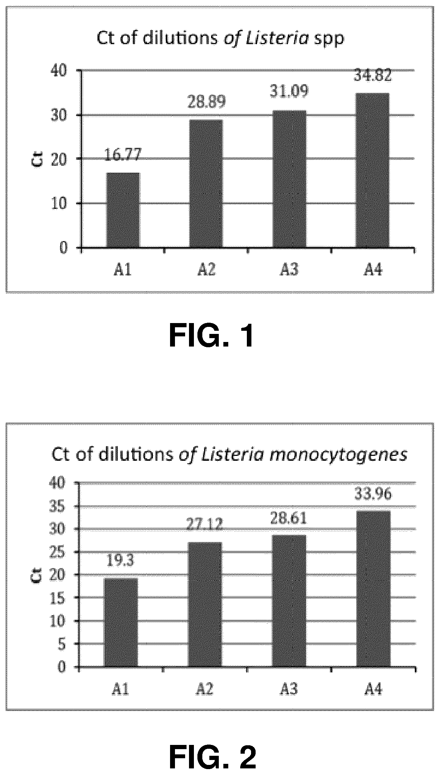

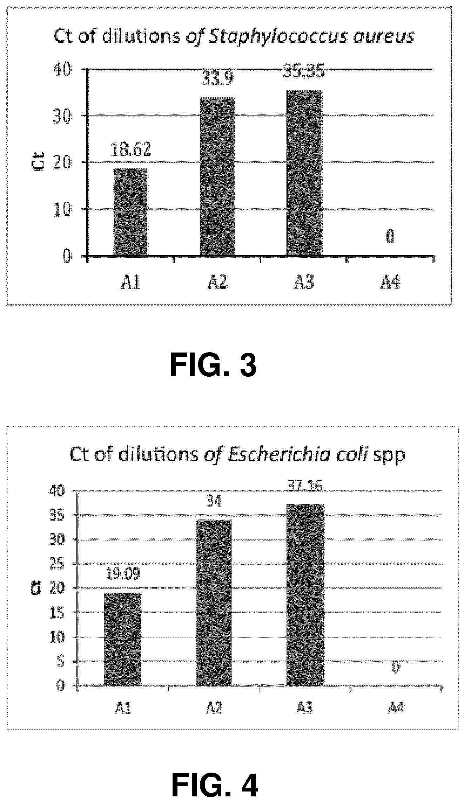

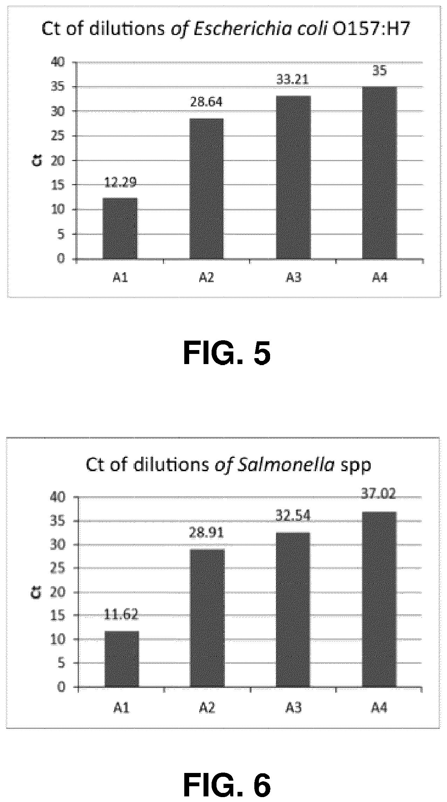

To determine the efficiency of DNA extraction, establish detection limits for each bacterium of interest and its quantification in non-enriched samples, calibration curves were performed with bacterial DNA with 3 serial dilutions (A2, A3, A4) from a 0.5 McFarland suspension (A1) of each of the study microorganisms. The results are observed in Figures 1 to 6. In each figure, the threshold cycle value (Ct) of fluorescence emission obtained for each sample is observed.

### DIAGNOSTIC KITS OF THE INVENTION

In another aspect, the present invention relates to a diagnostic kit useful for the implementation of the method of the invention regarding the multiple and simultaneous detection of any combination of Gram-positive and Gram-negative bacteria from among *Listeria* ssp., *Listeria monocytogenes, Staphylococcus aureus, Escherichia coli* spp., *Escherichia coli* O157:H7 y *Salmonella* spp. In general, the diagnostic kit of the present invention contains one or more pairs of oligonucleotide primers of the sequences SEQ ID NO: 1 and SEQ ID NO: 2, SEQ ID NO: 4 and SEQ ID NO: 5, SEQ ID NO: 7 and SEQ ID NO: 8, SEQ ID NO: 10 and SEQ ID NO: 11, SEQ ID NO: 13 and SEQ ID NO: 14, SEQ ID NO: 16 and SEQ ID NO: 17 corresponding to each of the bacteria Gram-positive and Gram-negative to detect *(Listeria* ssp., *Listeria monocytogenes, Staphylococcus aureus, Escherichia coli* spp., *Escherichia coli* O157:H7 y *Salmonella* spp.), respectively; likewise, the diagnostic kit has one or more of the TaqMan probes of sequences SEQ ID NO: 3, SEQ ID NO: 6, SEQ ID NO: 9, SEQ ID NO: 12, SEQ ID NO: 15 and SEQ ID NO: 18 for each of these Gram-positive and Gram-Negative bacteria, in order to be able to choose between detecting one or more bacteria from among *Listeria* ssp., *Listeria monocytogenes, Staphylococcus aureus, Escherichia coli* spp., *Escherichia coli* O157:H7 y *Salmonella* spp., depending on the needs. Alternatively, the diagnostic kit includes a pair of indicators of sequences SEQ ID NO: 19 and SEQ ID NO: 20, and a probe of sequence SEQ ID NO: 21 in order to be able to carry out an internal amplification control.

The diagnostic kit provided by this invention can be presented in the form of a kit containing, in addition to containers with the previously mentioned pairs of oligonucleotide primers and/or labeled probes, containers with all or part of the rest of the reagents necessary for carrying out. of the method in question, for example, ultrapure water, dNTPs (dATP, dCTP, dGTP, and dTTP), a suitable buffer for the enzymatic amplification reaction, a thermostable DNA polymerase (for example, Taq DNA polymerase), a magnesium salt (for example, MgCl2), among others. Additionally and optionally, the first diagnostic kit provided by this invention can include containers with DNA of *Listeria* ssp., *Listeria monocytogenes, Staphylococcus aureus, Escherichia coli* spp., *Escherichia coli* O157: H7 y *Salmonella* spp. for use as positive controls.

### EXAMPLES OF EMBODIMENT OF THE INVENTION

The invention will now be described with respect to the following examples, which are solely for the purpose of representing the manner of carrying out the implementation of the principles of the invention. The following examples are not intended to be an exhaustive representation of the invention, nor are they intended to limit the scope of the invention.

### EXAMPLE 1

### Materials and methods

### 1. Microbial strains

### 1.1 Collection of microorganisms, media and culture conditions.

A collection of 100 microorganisms has been generated, made up of foodborne pathogens and interfering bacteria that can be found both in food matrices and in the environment. The study strains used were *Listeria* spp. ATCC 33090, *Listeria monocytogenes* ATCC 19115, Salmonella enterica subsp. enteric serovar Typhimurium ATCC 14028, which were obtained in the form of lyophilized, reconstituted and cultured cultures following the recommended instructions. Likewise, the conditions and culture media used correspond to those recommended by the suppliers of the strains (Table 4). The strains of *Escherichia coli* spp., *Escherichia coli* O157:H7, *Salmonella* spp. and other bacteria phylogenetically close and distant to those studied (see Table 4) were isolated from food samples, purified and characterized by the Api miniaturized system (BioMérieux)

| Strain | Code | Culture Medium | Temperature °C |
|---|---|---|---|
| *Listeria* spp. | ATCC-33090 | Oxford agar | 35 |
| *Listeria monocytogenes* | ATCC-19115 | Oxford agar | 35 |
| *Staphylococcus aureus* | Sistema Api | Blood agar | 35 |
| *Escherichia coli* spp. | Sistema Api | Blood agar | 35 |
| *Escherichia coli O157:H7* | Sistema Api | Blood agar | 35 |
| *Salmonella* spp. | ATCC-14018 | Blood agar | 35 |
| *Staphylococcus epidermidis* | Sistema Api | Blood agar | 35 |
| *Staphylococcus saprophyticus* | Sistema Api | Blood agar | 35 |
| *Streptococcus faecalis* | Sistema Api | Blood agar | 35 |

**Table 4**

| | | | |
|---|---|---|---|
| *Listeria ivanovii* | Sistema Api | Oxford agar | 35 |
| *Serratia marcescens* | Sistema Api | Blood agar | 35 |
| *Citrobacter freundi* | Sistema Api | Blood agar | 35 |
| *Morganella morganii* | Sistema Api | Blood agar | 35 |
| *Lactobacillus rhamnosus* | Sistema Api | MRS agar | 30, CO₂ |
| *Enterococcus faecium* | Sistema Api | MRS agar | 30, CO₂ |
| *Pasteurella* spp. | Sistema Api | Blood agar | 35 |

The handling and manipulation of the strains was carried out in a Telstar BiollA level II biosafety laminar flow cabinet.

### 1.2 Conservation of cultures

The conservation of the strains in 20% glycerol at -80 °C has been used to guarantee cell viability, storing aliquots of each strain.

### 1.3. Artificial contamination of food matrices.

The food matrices used in the study are: meat products (sausages, hams), dairy products (fresh cheese, ripened cheese), frozen products (burritos, breaded products, weathered products), pizzas and meat raw materials (chorizo, bacon).

The samples of surfaces and environments were collected with an extended surface sponge.

For the artificial contamination of samples without enrichment, the 6 bacteria of interest were inoculated, 1) in individual tests and 2) in mixtures, with the same concentration and in different combinations: 0° UFC/mL, 10¹ UFC/mL, 10² UFC/mL, 10³ UFC/mL. The trials were also conducted with other phylogenetically close and distant bacteria. See Table 5. Subsequently, they were enriched in SEL medium. See Table 6. The tests were carried out in duplicate.

Prior to contamination of matrices, tests were carried out with individual strains and in mixtures, without enrichment and enriched in SEL medium, using the same mixture and concentration scheme described for the inoculation tests.

To carry out the assays with matrices without enrichment, 25 g of the sample were weighed into sterile mesh bags.

**Table 5**

| Food sample | Inocula | | | |
|---|---|---|---|---|
| Meat products (sausages, hams), dairy products (fresh cheese, matured cheese), Frozen (burritos, breaded, weathered), Pizzas, meat raw materials (chorizo, bacon) | *Listeria* spp. | *Listeria monocytogenes* | *Staphylococcus aureus* | *Listeria ivanovii, Staphylococcus epidermidis, Staphylococcus saprophyticus, Streptococcus faecalis, Lactobacillus rhamnosus, Enterococcus faecium* |
| | *Salmonella* s.pp | *Escherichia coli* | *Escherichia coli* O157: H7 | *Serratia marcescens, Citrobacter freundii, Morganella morganii, Pasteurella* spp. |

**Table 6**

| | | | |
|---|---|---|---|
| Concentration mixtures | *Listeria* ssp., *Listeria monocytogenes, Staphylococcus aureus* | *Salmonella* spp., *Escherichia coli, Escherichia coli* O157: H7 | *Listeria monocytogenes , Salmonella* spp., *Escherichia coli* O157: H7 |
| 10³:10³:10³ | | | |
| 10²:10²:10² | | | |
| 10¹:10¹:10¹ | | | |
| 10⁰:10⁰:10⁰ | | | |
| 10³:10⁰:10¹ | | | |
| 10⁰:10¹:10³ | | | |
| 10¹:10³:10⁰ | | | |

### 2. DNA collection and manipulation

### 2.1 Genomic DNA extraction.

### a) From pure cultures of standard strains.

For the tests with strains and inoculation of matrices, pure cultures were obtained from which 0.5 McFarland concentrates were prepared, corresponding to 10⁸ UFC/mL. A series of serial dilutions was prepared until obtaining concentrations of 10⁰ UFC/mL. DNA extraction was performed with Chelex, glass beads and mechanical disruption.

### b) From artificially contaminated food samples:

For DNA extraction from the food matrices indicated in Table 7, the following protocol was used:

### Sample 1: DNA sample preparation from inoculated and enriched food matrix samples:

1. Weigh 25 grams of the sample into a sterile mesh bag.
2. Enrich the samples with 225 mL of SEL enrichment medium.
3. Incubate at 35 °C +/- 2 °C for 24 hours +/- 2 hours.
4. Take two milliliters of the previously incubated broth and transfer it to a 2 mL tube.
5. Centrifuge at 14,000 rpm for 10 minutes.
6. Decant the supernatant.
7. Add 150 µl of thermal lysis buffer and 20 µl of a suspension with glass beads.
8. Disrupt the sample for 5 minutes.
9. Incubate for 10 minutes in a water bath at 95 °C.
10. Give a spin no longer than 30 seconds.
11. Transfer 30 µl of the supernatant to a 0.6 mL tube.
12. Safeguard.

### 3. Polymerase chain reaction (PCR)

### a) Uniplex

Uniplex PCR reactions were performed in a 7500 thermal cycler (Thermo Fisher) to confirm the obtaining of amplified DNA of the microorganisms under study from the cultures obtained from the inoculated food samples, surfaces and environments, as well as in pure or standard cultures.. For this, the initiators designed in reaction mixtures were used in uniplex mode under the conditions indicated by the manufacturer of the mixture that includes the polymerase enzyme.

### b) Multiplex

PCR was carried out in multiplex mode for the multiple detection of three Gram positive bacteria *(Listeria* ssp., *Listeria monocytogenes y Staphylococcus aureus),* using the designed primers. For this, two initial mixtures of initiators were prepared at a defined concentration, starting from the storage concentration of 5-20 µM of each one, and the concentrations of reagents and amplification conditions recommended by the manufacturer.

Likewise, multiplex PCR was carried out for the multiple detection of three Gram negative bacteria *(Escherichia coli* spp., *Escherichia coli* O157:H7, *Salmonella* spp.), Using the designed primers. For this, two initial mixtures of initiators were prepared at a defined concentration, starting from the storage concentration of 2.5-5 µM of each of them, and the concentrations of reagents and amplification conditions recommended by the manufacturer.

### 3.3. Internal Control Amplification (CIA)

To rule out false negatives in the detection and verify the correct functioning of the PCR reactions, an internal amplification control (CIA) was designed, consisting of a DNA sequence exogenous to the microorganisms used and to the sequences corresponding to the annealing sites of the primers designed for PCR reactions.

The exogenous sequence selected corresponds to a fragment of SEQ NO 19, SEQ NO 20 and SEQ NO 21. 50 pg of a 450 bp Gblock fragment containing the target sequence for the CIA was added to the tube with the multiplex reaction mixture.

The reaction conditions initially used are shown in Tables 7, 8, 9, 10, 11 and 12.

| Component of the Reaction Mixture | | Initial concentration | Final concentration in the reaction |
|---|---|---|---|
| Master mix | | | |
| *Listeria* spp. | SEQ ID NO: 1 | 1 | 100 |
| | SEQ ID NO: 2 | 1 | 100 |
| | SEQ ID NO: 3 | 0.25 | 25 |
| Water | | 2.25 | 225 |
| DNA test sample | | 5 | |

**Table 7**

| | Temperature (°C) | Time (s) | Cycles |
|---|---|---|---|
| Initial process | 94 | 120 | 1 |
| Amplification | 94 | 15 | 40 |
| | 63 | 25 | |

**Table 8**

| Component of the Reaction Mixture | | Initial concentration | Final concentration in the reaction |
|---|---|---|---|
| Master mix | | | |
| *Listeria monocytogenes* | SEQ ID NO: 4 | 1 | 100 |
| | SEQ ID NO: 5 | 1 | 100 |
| | SEQ ID NO: 6 | 0.25 | 25 |
| Water | | 2.25 | 225 |
| DNA test sample | | 5 | |

| | Temperature (°C) | Time (s) | Cycles |
|---|---|---|---|
| Initial process | 94 | 120 | 1 |
| Amplification | 94 | 15 | 40 |
| | 63 | 25 | |

**Table 9**

| Component of the Reaction Mixture | | Initial concentration | Final concentration in the reaction |
|---|---|---|---|
| Master mix | | | |
| *Staphylococcus aureus* | SEQ ID NO: 7 | 1 | 100 |
| | SEQ ID NO: 8 | 1 | 100 |
| | SEQ ID NO: 9 | 0.25 | 25 |
| Water | | 2.25 | 225 |
| DNA test sample | | 5 | |

| | Temperature (°C) | Time (s) | Cycles |
|---|---|---|---|
| Initial process | 94 | 120 | 1 |
| Amplification | 94 | 15 | 40 |
| | 63 | 25 | |

**Table 10**

| Component of the Reaction Mixture | | Initial concentration | Final concentration in the reaction |
|---|---|---|---|
| Master mix | | | |
| *Escherichia coli* spp. | SEQ ID NO: 10 | 1 | 100 |
| | SEQ ID NO: 11 | 1 | 100 |
| | SEQ ID NO: 12 | 0.25 | 25 |
| Water | | 2.25 | 225 |
| DNA test sample | | 5 | |

| | Temperature (°C) | Time (s) | Cycles |
|---|---|---|---|
| Initial process | 94 | 120 | 1 |
| Amplification | 94 | 15 | 40 |
| | 63 | 25 | |

**Table 11**

| Component of the Reaction Mixture | | Initial concentration | Final concentration in the reaction |
|---|---|---|---|
| Master mix | | | |
| *Escherichia coli* O157:H7 | SEQ ID NO: 13 | 1 | 100 |
| | SEQ ID NO: 14 | 1 | 100 |
| | SEQ ID NO: 15 | 0.25 | 25 |
| Water | | 2.25 | 225 |
| DNA test sample | | 5 | |

| | Temperature (°C) | Time (s) | Cycles |
|---|---|---|---|
| Initial process | 94 | 120 | 1 |
| Amplification | 94 | 15 | 40 |
| | 63 | 25 | |

**Table 12**

| Component of the Reaction Mixture | | Initial concentration | Final concentration in the reaction |
|---|---|---|---|
| Master mix | | | |
| *Salmonella* spp. | SEQ ID NO: 16 | 1 | 100 |
| | SEQ ID NO: 17 | 1 | 100 |
| | SEQ ID NO: 18 | 0.25 | 25 |
| Water | | 2.25 | 225 |
| DNA test sample | | 5 | |

| | Temperature (°C) | Time (s) | Cycles |
|---|---|---|---|
| Initial process | 94 | 120 | 1 |
| Amplification | 94 | 15 | 40 |
| | 63 | 25 | |

### d) Multiplex PCR

Multiplex mode PCR assays were carried out for the simultaneous detection of three Gram-positive bacteria *(Listeria* ssp., *Listeria monocytogenes y Staphylococcus aureus)* and three Gram-negative bacteria *(Escherichia coli* spp., *Escherichia coli* O157:H7, *Salmonella* spp.). To do this, the pair of primers and the specific TaqMan probe were included in each reaction for each of these three pathogens, also adding the CIA with its respective TaqMan probe, according to Tables 13 and 14.

**Table 13**

| Component of the Reaction Mixture | | Initial concentration | Amount to add (µl) | Final concentration |
|---|---|---|---|---|
| MgCl₂ | | 50 mmol | 1.75 | 3.5 |
| DNTPs | | 10 mmol | 0.5 | 200 µmol |
| Buffer | | 10X | 3.75 | 1.5X |
| *Listeria* spp. | SEQ ID NO: 1 | 5 nmol | 1 | 200 pmol |
| | SEQ ID NO: 2 | 5 nmol | 1 | 200 pmol |
| | SEQ ID NO: 3 | 5 nmol | 0.25 | 50 pmol |
| *Listeria monocytogenes* | SEQ ID NO: 4 | 5 nmol | 1 | 200 pmol |
| | SEQ ID NO: 5 | 5 nmol | 1 | 200 pmol |
| | SEQ ID NO: 6 | 5 nmol | 0.25 | 50 pmol |
| *Staphylococcus aureus* | SEQ ID NO: 7 | 5 nmol | 1.5 | 300 pmol |
| | SEQ ID NO: 8 | 5 nmol | 1.5 | 200 pmol |
| | SEQ ID NO: 9 | 5 nmol | 1.75 | 350 pmol |
| Taq DNA polymerase | | 5 U/µl | 0.25 | |
| Water | | | 2.25 | |
| DNA test sample | | | 5 | |
| Total volume | | | 25 | |

**Table 14**

| Component of the Reaction Mixture | | Initial concentration | Amount to add (µl) | Final concentration |
|---|---|---|---|---|
| MgCl₂ | | 50 mmol | 1.75 | 3,5 |
| DNTPs | | 10 mmol | 0.5 | 200 µmol |
| Buffer | | 10X | 3.75 | 1,5X |
| *Escherichia coli* spp. | SEQ ID NO: 10 | 5 nmol | 1 | 200 pmol |
| | SEQ ID NO: 11 | 5 nmol | 1 | 200 pmol |
| | SEQ ID NO: 12 | 5 nmol | 0.25 | 50 pmol |
| *Escherichia coli* O157:H7 | SEQ ID NO: 13 | 5 nmol | 1 | 200 pmol |
| | SEQ ID NO: 14 | 5 nmol | 1 | 200 pmol |
| | SEQ ID NO: 15 | 5 nmol | 0.25 | 50 pmol |
| *Salmonella* spp. | SEQ ID NO: 16 | 5 nmol | 1.5 | 300 pmol |
| | SEQ ID NO: 17 | 5 nmol | 1.5 | 200 pmol |
| | SEQ ID NO: 18 | 5 nmol | 1.75 | 350 pmol |
| Taq DNA polymerase | | 5 U/µl | 0.25 | |
| Water | | | 2,25 | |
| DNA test sample | | | 5 | |
| Total volume | | | 25 | |

Subsequently, a reaction cocktail is prepared for 100 reactions by mixing the components described in Tables 15 and 16.

**Table 15**

| Component of the Reaction Mixture | | Amount to add (µl) for one reaction | Amount to add (µl) for 100 reactions |
|---|---|---|---|
| MgCl₂ | | 1.75 | 175 |
| DNTPs | | 0.5 | 50 |
| Buffer | | 3.75 | 375 |
| *Listeria* spp. | SEQ ID NO: 1 | 1 | 100 |
| | SEQ ID NO: 2 | 1 | 100 |
| | SEQ ID NO: 3 | 0.25 | 25 |
| *Listeria monocytogenes* | SEQ ID NO: 4 | 1 | 100 |
| | SEQ ID NO: 5 | 1 | 100 |
| | SEQ ID NO: 6 | 0.25 | 25 |
| *Staphylococcus aureus* | SEQ ID NO: 7 | 1.5 | 150 |
| | SEQ ID NO: 8 | 1.5 | 150 |
| | SEQ ID NO: 9 | 1.75 | 175 |
| Water | | 2.25 | 225 |
| DNA test sample | | 5 | |

**Table 16**

| Component of the Reaction Mixture | | Amount to add (µl) for one reaction | Amount to add (µl) for 100 reactions |
|---|---|---|---|
| MgCl₂ | | 1.75 | 175 |
| DNTPs | | 0.5 | 50 |
| Buffer | | 3.75 | 375 |
| *Escherichia coli* spp. | SEQ ID NO: 10 | 1 | 100 |
| | SEQ ID NO: 11 | 1 | 100 |
| | SEQ ID NO: 12 | 0.25 | 25 |
| *Escherichia coli* O157:H7 | SEQ ID NO: 13 | 1 | 100 |
| | SEQ ID NO: 14 | 1 | 100 |
| | SEQ ID NO: 15 | 0.25 | 25 |
| *Salmonella* spp. | SEQ ID NO: 16 | 1.5 | 150 |
| | SEQ ID NO: 17 | 1.5 | 150 |
| | SEQ ID NO: 18 | 1.75 | 175 |
| Water | | 2.25 | 225 |
| DNA test sample | | 5 | |

### RESULTS

In an exemplary embodiment, Table 17 indicates the conditions in which each multiplex amplification reaction was carried out with a different diagnostic kit, Table 6 indicates the detection limits reached depending on the diagnostic kit and shows the readings of the concentrations that were obtained as a result of the multiple and simultaneous detection of the three Gram-positive bacteria *(Listeria* ssp., *Listeria monocytogenes y Staphylococcus aureus)* and/or of the three Gram-negative bacteria *(Escherichia coli* spp., *Escherichia coli* O157:H7 y *Salmonella* spp.) in food and environment samples, by using a first kit with a single mixture of reagents and three specific oligonucleotides for different Gram-positive bacteria at a single temperature condition and mixing for their amplification, or by using of a second kit with a single reagent mix and three specific oligonucleotides for different Gram-negative bacteria to a single condition of temperatures and mixing for amplification.

**Table 17**

| | Temperature (°C) | Time (s) | Cyclos |
|---|---|---|---|
| Initial process | 94 | 120 | 1 |
| Amplification | 94 | 15 | 40 |
| | 63 | 25 | |

Based on the embodiments described above, it is contemplated that modifications to the described environments of performance, as well as alternative environments of performance will be considered apparent to a person skilled in the art under the present disclosure. It is therefore contemplated that the claims encompass such modifications and alternatives that are within the scope of the present invention or its equivalents.

## Claims

1. A method for detecting multiple and simultaneously a combination of Gram-positive bacteria and/or Gram-negative bacteria in a sample, the method comprises the steps of:
a) providing DNA extracted from the sample;
b) preparing a reaction mixture, wherein the reaction mixture includes:
the DNA;
one or more pairs of oligonucleotide primers having a sequence selected from the group consisting of SEQ ID NO: 1 and SEQ ID NO: 2, SEQ ID NO: 4 and SEQ ID NO: 5, SEQ ID NO: 7 and SEQ ID NO: 8, SEQ ID NO: 10 and SEQ ID NO: 11, SEQ ID NO: 13 and SEQ ID NO: 14, SEQ ID NO: 16 and SEQ ID NO: 17 and combinations thereof; and
and one or more oligonucleotide probes having a sequence selected from the group consisting of SEQ ID NO: 3, SEQ ID NO: 6, SEQ ID NO: 9, SEQ ID NO: 12, SEQ ID NO: 15, SEQ ID NO: 18 and combinations thereof;
c) amplifying the DNA included in the reaction mixture, by means real-time polymerase chain reaction; and
d) simultaneously determining the presence or absence of Gram-positive bacteria and/or Gram-negative bacteria in the sample.

2. The method of claim 1, wherein the probes are designed to amplify target nucleic acid of one or more of *Listeria* ssp., *Listeria monocytogenes, Staphylococcus aureus, Escherichia coli* spp., *Escherichia coli* O157: H7 and *Salmonella* spp.

3. The method of claim 1, wherein each oligonucleotide probe of sequence SEQ ID NO: 3, SEQ ID NO: 6, SEQ ID NO: 9, SEQ ID NO: 12, SEQ ID NO: 15, SEQ ID NO : 18 is marked at its 5'-end with a fluorophore and at its 3'-end with an extinguisher.

4. The method of claim 3, wherein the fluorophore for the oligonucleotide probe of sequence SEQ ID NO: 3 is VIC.

5. The method of claim 3, wherein the extinguisher for the oligonucleotide probe of sequence SEQ ID NO: 3 is MGB.

6. The method of claim 3, wherein the fluorophore for the oligonucleotide probe of sequence SEQ ID NO: 6 is FAM.

7. The method of claim 3, wherein the extinguisher for the oligonucleotide probe of sequence SEQ ID NO: 6 is MGB.

8. The method of claim 3, wherein the fluorophore for the oligonucleotide probe of sequence SEQ ID NO: 9 is JUN.

9. The method of claim 3, wherein the extinguisher for the oligonucleotide probe of sequence SEQ ID NO: 9 is QSY.

10. The method of claim 3, wherein the fluorophore for the oligonucleotide probe of sequence SEQ ID NO: 12 is VIC.

11. The method of claim 3, wherein the extinguisher for the oligonucleotide probe of sequence SEQ ID NO: 15 is MGB.

12. The method of claim 3, wherein the fluorophore for the oligonucleotide probe of sequence SEQ ID NO: 18 is FAM.

13. The method of claim 3, wherein the extinguisher for the oligonucleotide probe of sequence SEQ ID NO: 12 is MGB.

14. The method of claim 3, wherein the fluorophore for the oligonucleotide probe of sequence SEQ ID NO: 15 is JUN.

15. The method of claim 3, wherein the extinguisher for the oligonucleotide probe of sequence SEQ ID NO: 18 is QSY.

16. The method of claim 1, wherein further includes the step of performing an internal amplification control with a pair of oligonucleotide primers of sequence SEQ ID NO: 19 and SEQ ID NO: 20 and an oligonucleotide probe of sequence SEQ ID NO: 21.

17. A diagnostic kit for detecting multiple and simultaneously a combination of Gram-positive bacteria and/or Gram-negative bacteria in a simple, the diagnostc kit comprising:
one or more pairs of oligonucleotide primers having a sequence selected from the group consisting of SEQ ID NO: 1 and SEQ ID NO: 2, SEQ ID NO: 4 and SEQ ID NO: 5, SEQ ID NO: 7 and SEQ ID NO: 8, SEQ ID NO: 10 and SEQ ID NO: 11, SEQ ID NO: 13 and SEQ ID NO: 14, SEQ ID NO: 16 and SEQ ID NO: 17 and combinations thereof; and
one or more oligonucleotide probes having a sequence selected from the group consisting of SEQ ID NO: 3, SEQ ID NO: 6, SEQ ID NO: 9, SEQ ID NO: 12, SEQ ID NO: 15, SEQ ID NO: 18 and combinations thereof.

18. The diagnostic kit of claim 17, wherein the probes are designed to amplify target nucleic acid of one or more of *Listeria* ssp., *Listeria monocytogenes, Staphylococcus aureus, Escherichia coli* spp., *Escherichia coli* O157: H7 and *Salmonella* spp.

19. The diagnostic kit of claim 17, wherein each oligonucleotide probe of sequence SEQ ID NO: 3, SEQ ID NO: 6, SEQ ID NO: 9, SEQ ID NO: 12, SEQ ID NO: 15, SEQ ID NO : 18 is marked at its 5'-end with a fluorophore and at its 3'-end with an extinguisher.

20. The diagnostic kit of claim 19, wherein the fluorophore for the oligonucleotide probe of sequence SEQ ID NO: 3 is VIC.

21. The diagnostic kit of claim 19, wherein the extinguisher for the oligonucleotide probe of sequence SEQ ID NO: 3 is MGB.

22. The diagnostic kit of claim 19, wherein the fluorophore for the oligonucleotide probe of sequence SEQ ID NO: 6 is FAM.

23. The diagnostic kit of claim 19, wherein the extinguisher for the oligonucleotide probe of sequence SEQ ID NO: 6 is MGB.

24. The diagnostic kit of claim 19, wherein the fluorophore for the oligonucleotide probe of sequence SEQ ID NO: 9 is JUN.

25. The diagnostic kit of claim 19, wherein the extinguisher for the oligonucleotide probe of sequence SEQ ID NO: 9 is QSY.

26. The diagnostic kit of claim 19, wherein the fluorophore for the oligonucleotide probe of sequence SEQ ID NO: 12 is VIC.

27. The diagnostic kit of claim 19, wherein the extinguisher for the oligonucleotide probe of sequence SEQ ID NO: 15 is MGB.

28. The diagnostic kit of claim 19, wherein the fluorophore for the oligonucleotide probe of sequence SEQ ID NO: 18 is FAM.

29. The diagnostic kit of claim 19, wherein the extinguisher for the oligonucleotide probe of sequence SEQ ID NO: 12 is MGB.

30. The diagnostic kit of claim 19, wherein the fluorophore for the oligonucleotide probe of sequence SEQ ID NO: 15 is JUN.

31. The diagnostic kit of claim 19, wherein the extinguisher for the oligonucleotide probe of sequence SEQ ID NO: 18 is QSY.

32. The diagnostic kit of claim 17, wherein further includes a pair of oligonucleotide primers of sequence SEQ ID NO: 19 and SEQ ID NO: 20 and an oligonucleotide probe of sequence SEQ ID NO: 21 for performing an internal amplification control.


SEQUENCE LISTING

<110>  SIGMA ALIMENTOS, S. A. DE C. V.

<120>  DIAGNOSTIC KIT AND METHOD FOR THE SIMULTANEOUS DETECTION OF A
       COMBINATION OF MULTIPLE GRAM-POSITIVE BACTERIA AND/OR
       GRAM-NEGATIVE BACTERIA

<130>  SIGMA073PAT

<160>  21

<170>  PatentIn version 3.5

<210>  1
<211>  20
<212>  DNA
<213>  Artificial

<220>
<223>  Oligonucleotide "upstream" of Listeria ssp

<400>  1
atagggaatc gcacgaatgg                                                   20


<210>  2
<211>  20
<212>  DNA
<213>  Artificial

<220>
<223>  Oligonucleotide "downstream" of Listeria ssp

<400>  2
agcggatttg cctacttctc                                                   20


<210>  3
<211>  24
<212>  DNA
<213>  Artificial

<220>
<223>  Oligonucleotide probe of Listeria ssp

<400>  3
aaatgtgcgt ccaagcagtg agtg                                              24


<210>  4
<211>  22
<212>  DNA
<213>  Artificial

<220>
<223>  Oligonucleotide "upstream" of Listeria monocytogenes

<400>  4
ctcatttcac atcgtccatc ta                                                22


<210>  5

<211>  19
<212>  DNA
<213>  Artificial

<220>
<223>  Oligonucleotide "downstream" of Listeria monocytogenes

<400>  5
gttctccacc attcccaag                                                    19


<210>  6
<211>  25
<212>  DNA
<213>  Artificial

<220>
<223>  Oligonucleotide probe of Listeria monocytogenes

<400>  6
tgtttacgct aaagaatgca ctggt                                             25


<210>  7
<211>  25
<212>  DNA
<213>  Artificial

<220>
<223>  Oligonucleotide "upstream" of Staphylococcus aureus

<400>  7
tgatacacct gaaacaaagc atcct                                             25


<210>  8
<211>  33
<212>  DNA
<213>  Artificial

<220>
<223>  Oligonucleotide "downstream" of Staphylococcus aureus

<400>  8
gacttcaatt ttctttgcat tttctaccat ttt                                    33


<210>  9
<211>  16
<212>  DNA
<213>  Artificial

<220>
<223>  Oligonucleotide probe of Staphylococcus aureus

<400>  9
cagggccata tttctc                                                       16


<210>  10
<211>  19
<212>  DNA
<213>  Artificial

<220>
<223>  Oligonucleotide "upstream" of Escherichia coli spp

<400>  10
ggcaaagtgt gggtcaata                                                    19


<210>  11
<211>  21
<212>  DNA
<213>  Artificial

<220>
<223>  Oligonucleotide "downstream" of Escherichia coli spp

<400>  11
gatagtctgc cagttcagtt c                                                 21


<210>  12
<211>  24
<212>  DNA
<213>  Artificial

<220>
<223>  Oligonucleotide probe of Escherichia coli spp

<400>  12
aagccgatgt cacgccgtat gtta                                              24


<210>  13
<211>  23
<212>  DNA
<213>  Artificial

<220>
<223>  Oligonucleotide "upstream" of Escherichia coli 0157:H7

<400>  13
cttacgcttc aggcagatac aga                                               23


<210>  14
<211>  22
<212>  DNA
<213>  Artificial

<220>
<223>  Oligonucleotide "downstream" of Escherichia coli 0157:H7

<400>  14
cacaggagca gtttcagaca gt                                                22


<210>  15
<211>  15
<212>  DNA
<213>  Artificial

<220>
<223>  Oligonucleotide probe of Escherichia coli 0157:H7

<400>  15
cctgacgaaa ttctc                                                        15


<210>  16
<211>  23
<212>  DNA
<213>  Artificial

<220>
<223>  Oligonucleotide "upstream" of Salmonella spp.

<400>  16
gtcctaacga cgacccttct ttt                                               23


<210>  17
<211>  23
<212>  DNA
<213>  Artificial

<220>
<223>  Oligonucleotide "downstream" of Salmonella spp.

<400>  17
gccaaaccta aaaccagcaa agg                                               23


<210>  18
<211>  16
<212>  DNA
<213>  Artificial

<220>
<223>  Oligonucleotide probe of Salmonella spp.

<400>  18
cagccgctca gtattg                                                       16


<210>  19
<211>  21
<212>  DNA
<213>  Artificial

<220>
<223>  Oligonucleotide "downstream" of Internal Control Amplification

<400>  19
ccgaccaaat ccaaccagta a                                                 21


<210>  20
<211>  22
<212>  DNA
<213>  Artificial

<220>
<223>  Oligonucleotide "downstream" of Internal Control Amplification

<400>  20
ctggagagga tgagtgagaa gt                                                22

<210>  21
<211>  24
<212>  DNA
<213>  Artificial

<220>
<223>  Oligonucleotide probe of Internal Control Amplification

<400>  21
ccaaagccac ctgaaccaac tcct                                              24